# EUROPÄISCHE PATENTSCHRIFT

(11) **EP 1 966 118 B1**
(45) Veröffentlichungstag und Bekanntmachung des Hinweises auf die Patenterteilung: **11.03.2009**
(21) Anmeldenummer: 06829483.4
(22) Anmeldetag: 11.12.2006
(51) Int. Cl.: C07C 69/74, C07D 209/52

(54) **VERBESSERTES VERFAHREN ZUR HERSTELLUNG VON RAMIPRIL**
IMPROVED METHOD FOR THE PRODUCTION OF RAMIPRIL
PROCEDE AMELIORE DE PRODUCTION DE RAMIPRIL

(30) Priorität: 21.12.2005 DE 102005061756
(43) Veröffentlichungstag der Anmeldung: 10.09.2008
(73) Patentinhaber: Sanofi-Aventis Deutschland GmbH, 65929 Frankfurt am Main (DE)
(72) Erfinder: BERK, Holger, 65926 Frankfurt am Main (DE); ZOCHER, Frank, 65926 Frankfurt am Main (DE); FLEMMING, Hans-Wolfram, 65926 Frankfurt am Main (DE); GAULER, Rainer, 65926 Frankfurt am Main (DE); LEHNERT, Rudolf, 65926 Frankfurt am Main (DE); LAUX, Wolfgang, F-92165 Antony (FR)
(86) Internationale Anmeldenummer: PCT/EP2006/011891
(87) Internationale Veröffentlichungsnummer: WO 2007/079871

(56) Entgegenhaltungen:
- EP-A- 1 502 914
- WO-A-20/05049567
- WO-A-20/05049568
- US-A- 5 061 722
- CARBONI C ET AL: "Quantitative enzymatic protection of d-amino acid methyl esters by exploiting 'relaxed' enantioselectivity of penicillin-G amidase in organic solvent" TETRAHEDRON LETTERS, ELSEVIER, AMSTERDAM, NL, Bd. 45, Nr. 52, 20. Dezember 2004 (2004-12-20), Seiten 9649-9652, XP004727421 ISSN: 0040-4039

## Beschreibung

Die vorliegende Erfindung betrifft ein verbessertes Verfahren zur Herstellung von Ramipril, sowie in dem Verfahren verwendete Zwischenprodukte und deren Verwendung.

Ramipril ist ein häufig in der Pharmazie eingesetzter ACE-Inhibitor. Die bisher publizierten Verfahren zur Herstellung von Ramipril (siehe z.B. US-Patent Nr. 5,061,722) sind sehr aufwändig. Spezielle Verfahren zur Herstellung von ACE-Inhibitoren sind bekannt (siehe z.B. europäische Patentanmeldungen EP 0215335 und EP 0967221), führen aber zu anderen ACE-Inhibitoren oder bieten keine Lösungen hinsichtlich enantiomeren-spezifischer Synthese. In der Absicht, ein verbessertes Verfahren zur Herstellung von Ramipril bereitzustellen, wurde nun überraschenderweise ein Verfahren gefunden, bei dem mit einfachen Mitteln die Herstellung von Ramipril verbessert werden kann.

Die Erfindung betrifft demzufolge ein Verfahren zur Herstellung von Ramipril, einer Verbindung der formel (I) 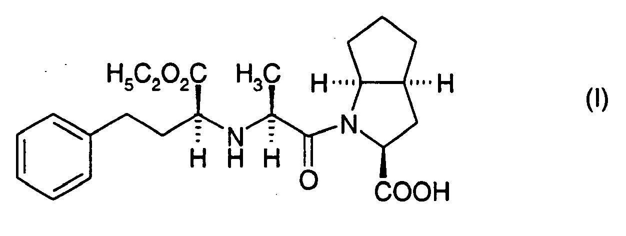 dadurch gekennzeichnet, dass
(A) eine Verbindung der Formel (II) 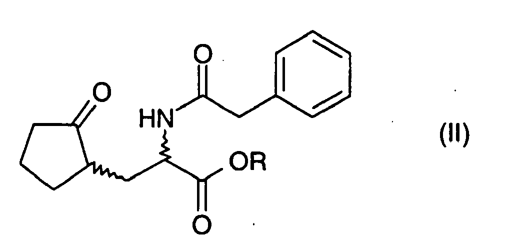 in der R (C₁-C₄)-Alkyl bedeutet, in einem geeigneten Lösungsmittel unter Zusatz von einer oder mehreren Basen oder Säuren zu einer Verbindung der Formel (III) 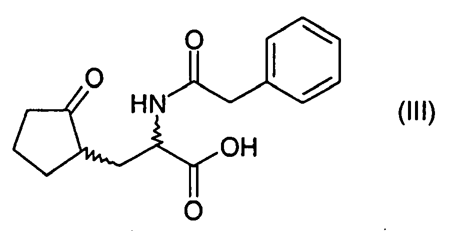 verseift wird, und anschließend
(B) die Verbindung der Formel (III) unter Einfluss von Penicillin-G-Amidase zu einem Gemisch der Verbindungen der Formeln (IV) und (V) 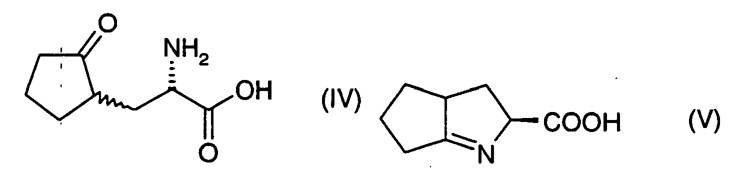 oder einem Gemisch von Salzen der Verbindungen der Formel (IV) und (V) umgesetzt wird, welche miteinander im Gleichgewicht stehen, und anschließend
(C) die Verbindung (V) oder ein Salz der Verbindung (V) aus dem Gemisch der Verbindungen (IV) und (V) oder deren Salzen durch katalytische Hydrierung zu einer Verbindung der Formel (VI) 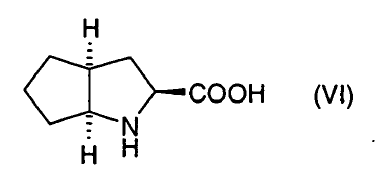 oder einem Salz davon umgesetzt wird, und anschließend entweder
(D-A) die Verbindung der Formel (VI) mit einer Verbindung der Formel (VII) 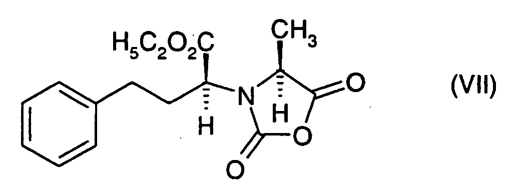 zur Verbindung der Formel (I) umgesetzt wird,
   oder alternativ
(D-B.1) Verbindung (VI) mit Benzylalkohol zu einer Verbindung der (VIII) 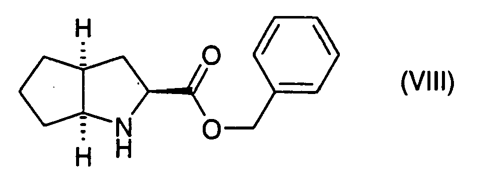 oder einem Salz davon umgesetzt wird, und anschließend
(D-B.2.1) Verbindung (VIII) entweder mit einer Verbindung der Formel (VII) zu einer Verbindung der Formel (X) 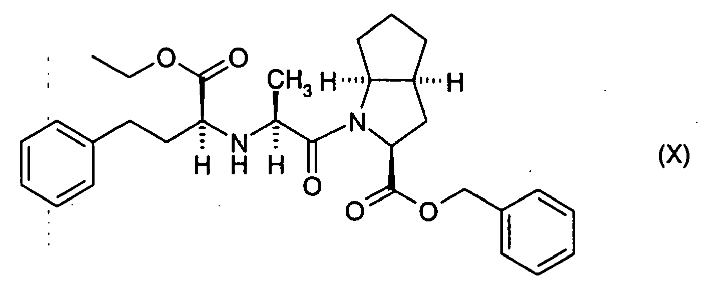 umgesetzt wird, oder alternativ
(D-B.2.2) die Verbindung (VIII) mit einer Verbindung der Formel (IX) 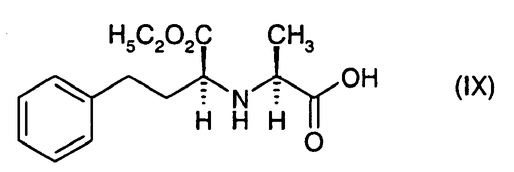 zur Verbindung (X) umgesetzt wird, und anschließend
(D-B.3) aus der Verbindung (X) durch katalytische Hydrierung die Verbindung der Formel (I) gebildet wird.
(C₁-C₄)-Alkyl bedeutet Methyl, Ethyl, n-Propyl, iso-Propyl, n-Butyl, iso-Butyl oder tert-Butyl.

Besonders vorteilhafte Teilschritte des erfindungsgemäßen Verfahrens bestehen in den Schritten (B) und (BA'), in denen durch den Einsatz von Penicillin-G-Amidase eine Verbindung mit einem racemischen Strukturelement in ein Reaktionsprodukt mit einem entsprechenden isomerenreinen Strukturelement umgewandelt wird. Diese Teilschritte sind jeweils ebenfalls Gegenstand der vorliegenden Erfindung.

Ein weiterer Gegenstand der vorliegenden Erfindung ist ein Verfahren zur Herstellung der Verbindung (I) ausgehend von der Verbindung (III) umfassend die Teilschritte (B), (C) und entweder (D-A) oder alternativ (D-B.1), (D.B.2.1) oder (D-B.2.2) und (D-B.3) wie oben definiert.

Ein weiterer Gegenstand der vorliegenden Erfindung ist das Zwischenprodukt der Formel (II) 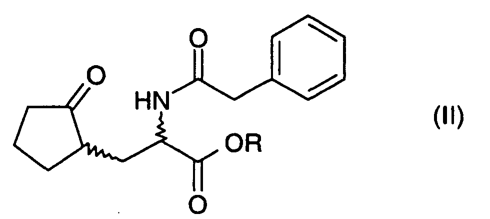 wobei R (C₁-C₄)-Alkyl bedeutet. Von besonderer Bedeutung ist die Verbindung der Formel (II), in der R CH₃ bedeutet. Die Herstellung der Verbindung der Formel (II), in der R Methyl bedeutet, kann beispielsweise erfolgen durch (1.) Umsetzung von Serin-methylester-hydrochlorid mit Phenylacetylchlorid in Gegenwart einer Base, z.B. Na₂CO₃, in einem geeigneten Lösungsmittel, z.B. Essigester und/oder Diisopropylether, zu N-Phenylacetyl-serin-methylester, beispielsweise nach Couloigner et al. (Bioorg. & Med. Chem. Lett. 1999, 9, 2205-2206), (2.) Chlorierung der so erhaltenen Verbindung unter Standardbedingungen, beispielsweise mit PCl₃ oder Thionylchlorid in einem geeigneten Lösungsmittel, z.B. Essigester, beispielsweise nach Anderson et al. (Synthesis 1976, 398-399), und (3.) Umsetzung des so erhaltenen 3-Chlor-2-phenylacetylamino-propionsäuremethylesters mit 1-Cyclopent-1-enyl-pyrrolidin (CAS Registry Nr. 7148-07-4) in Gegenwart einer Base, z.B. NEt₃, in einem geeigneten Lösungsmittel, z.B. Essigester und anschließender saurer Aufarbeitung mit z.B. HCl in Essigester, beispielsweise nach Teetz et al., Tetrahedron Lett. 1984, 25(40), 4479-4482. Alternativ kann die Verbindung der Formel (II), in der R Methyl bedeutet, beispielsweise herstellen durch (1.) Chlorierung von Serin-methylester-hydtochlorid mit beispielsweise PCl₅ in einem geeigneten Lösungsmittel, z.B. CH₂Cl₂, (2.) Umsetzung des so erhaltenen 2-Amino-3-chloro-propionsäuremethylester-hydrochlorids mit Phenylacetylchlorid in einem geeigneten Lösungsmittel, z.B. Toluen, und (3.) Umsetzung des so erhaltenen 3-Chloro-2-phenylacetylamino-propionsäure-methyesters mit 1-Cyclopent-1-enyl-pyrrolidin (CAS Registry Nr. 7148-07-4) in Gegenwart einer Base, z.B. NEt₃, in einem geeigneten Lösungsmittel, z.B. Essigester und anschließender saurer Aufarbeitung mit z.B. HCl in Essigester, beispielsweise nach Teetz et al., Tetrahedron Lett. 1984, 25(40), 4479-4482. Die übrigen Ester lassen sich analog zu den oben genannten Vorschriften herstellen oder sind durch Umesterung des Methylesters erhältlich. Vorzugsweise liegt Verbindung (II) als Gemisch von zwei diasteomeren Racematen vor.

Ein weiterer Gegenstand der vorliegenden Erfindung ist das Zwischenprodukt der Formel (III)

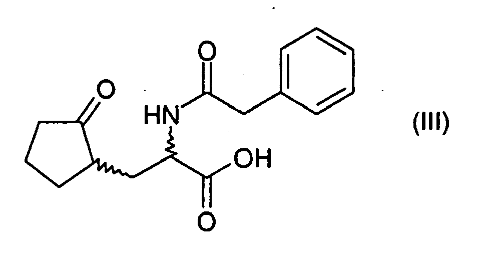

Die Verbindung der Formel (III) wird in Schritt (A) durch Verseifung einer Verbindung der Formel (II) hergestellt. Dies kann nach dem Fachmann bekannten Methoden erfolgen, beispielsweise indem die Verbindung der Formel (II) in einem geeigneten Lösungsmittel vorgelegt wird und zunächst eine oder mehrere Basen, beispielsweise NaOH und/oder KOH, vorzugsweise NaOH hinzugefügt wird und anschließend durch Zugabe von Säure, vorzugsweise HCl, ein pH-Wert im Bereich von 6 bis 7 eingestellt wird. Alternativ kann zunächst mittels einer oder mehrerer Säuren, beispielsweise Salzsäure, Bromwasserstoffsäure, Schwefelsäure oder Methansulfonsäure, vorzugsweise Salzsäure verseift, und anschließend durch Zugabe von Lauge ein pH-Wert von 6-7 eingestellt werden. Geeignete Lösungsmittel sind Wasser oder Wasser im Gemisch mit einem organischen Lösungsmitteln wie beispielsweise THF oder Dioxan. Es ist möglich, die Verbindung der Formel (III) zu isolieren. Bei dem erfindungsgemäßen Verfahren zur Herstellung der Verbindung der Formel (I) ist jedoch eine Weiterverarbeitung der Verbindung der Formel (III) *in situ* bevorzugt. Vorzugsweise liegt Verbindung (III) als Gemisch von zwei diasteomeren Racematen vor.

Schritt (B) des e'rfindungsgemäßen Verfahren kann erfolgen, indem die Verbindung der Formel (III) in wässriger Lösung vorgelegt wird und Penicillin-G-Amidase (E.C. 3.5.1.11) zugegeben wird. Das Enzym Penicillin-G-Amidase wird zur industriellen Herstellung von semisynthetischen Penicillin-Antibiotioka verwendet, wobei es die Hydrolyse von Penicillin G zum 6-Aminopenicillansäure (6-APA) und Phenylessigsäure katalysiert. Penicillin-G-Amidase wird ferner bei der Herstellung von Cephalosporin-Antibiotika verwendet, wobei es die Hydrolyse von Cephalosporin G zu 7-Aminodeacetoxycephalosporansäure (7-ADCA) und Phenylessigsäure katalysiert. Das Enzym kann immobilisiert oder nicht-immobilisiert eingesetzt werden, vorzugsweise wird ein auf einem Trägermaterial immobilisiertes Enzym eingesetzt. Dem Fachmann sind eine Vielzahl von möglichen Trägermaterialien bekannt, beispielsweise aminoaktivierte Silikate (Burteau et al., FEBS Letters (1989), 258(2), 185-189), DEAE-Cellulose (Warburton et al., Biochimica et Biophysica Acta, Enzymology (1972), 284(1), 278-84), Polyurethan (französische Patentanmeldung FR 2371470), makroporöse Träger (deutsche Patentanmeldung DE 3515252) oder "aminofunktionelle Organosiloxanpolymere" (US-Patent Nr. 5,780,260). Vorzugsweise wird in dem erfindungsgemäßen Verfahren die laut US 5,780,260 hergestellte, auf aminofunktionellen Organosiloxanpolymeren immobilisierte Penicillin-G-Amidase verwendet. Das Gewichtsverhältnis von Verbindung der Formel (III) zu immobilisierter Penicillin-G-Amidase sollte dabei im Verhältnis 5:1 bis 3:1 liegen (z.B. PGA 450 von Roche Diagnostics, Bestellnummer 1414208; ebenfalls möglich sind z.B. PGA Beads von Recordati oder Fermase PA 1500 bzw. Fermase PA 750 von Fermenta Biotech Ltd. (Indien)), wobei sich das Gewicht der Amidase auf das Enzym in polymergebundener Form bezieht. Alternativ kann auch eine Penicillin-G-Amidase der Firma FLUKA verwendet werden (Bestellnummer 76428: Penicillin G Amidase, immobilisiert von E. coli, ~150 U/g als feuchtes Material, wobei 1 U der Menge an Enzym entspricht, die 1 µmol Benzylpenicillin pro Minute bei pH 7,6 und 37°C hydrolysiert). Vorzugsweise liegt das Gewichtsverhältnis bei 4:1. Die Penicillin-G-Amidase kann dabei auch in Lösung bzw. Suspension (z.B. von Roche Diagnostics, Bestellnummer 1290959) oder in quervernetzten Zellen (z.B. Fermase PA 250 von Fermenta Biotech Ltd. (Indien)) vorliegen: Verfahrensschritt (B) kann bei unterschiedlichen Temperaturen durchgeführt werden. Bevorzugt ist eine Temperatur von 20 bis 45 °C, besonders bevorzugt eine Temperatur vom 26 bis 30 °C, insbesondere von ca. 28 °C. Die Reaktion kann bei pH-Werten zwischen 5 und 9.5 durchgeführt werden. Bevorzugt ist ein pH-Wert zwischen 6 und 7, besonders bevorzugt ist ein pH-Wert von 6,4. Als Lösungsmittel eignet sich Wasser oder Wasser im Gemisch mit einem (C₁-C₃)Alkanol, vorzugsweise Wasser im Gemisch mit Methanol, Ethanol oder iso-Propanol, oder Aceton, oder DMSO. Bevorzugt sind Gemische von Wasser mit iso-Propanol (50 % w/v), Aceton (35 % w/v), Ethanol (30 % w/v), Methanol (40 % w/v) oder DMSO (50 % w/v), jeweils bezogen auf die Endkonzentration.

Nach Beendigung der Reaktion wird dem Reaktionsgemisch ein organisches Lösungsmittel zugefügt, mit dem die ungewünschten Diastereomere extrahiert werden, wobei die gewünschten Diastereomere in der wässrigen Phase verbleiben. Die wässrige Phase hat vorzugsweise einen pH-Wert, der kleiner als oder gleich dem isoelektrischen Punkt des Salzes der aufzureinigenden Verbindung (IV) bzw. (V) ist. Als Lösungsmittel für die Extraktion eignen sich dem Fachmann bekannte mit Wasser schwer oder nicht mischbare Lösungsmittel. Mit Wasser schwer oder nicht mischbare Lösungsmittel sind zum Beispiel Ethylacetat, Propylacetat, Butylacetat, C₄-C₁₀-Alkanole, z.B. n-Butanol, Methylethylketon, Toluen, Diisopropylether oder Heptan. Ein besonders geeignetes Lösungsmittel ist Ethylacetat. Die Extraktion des ungewünschten isomers erfolgt vorzugsweise nach Zugabe von Säure, vorzugsweise Salzsäure. Der pH-Wert kann dabei auf einen Wert zwischen 0,5 und 5, vorzugsweise 1 und 4 eingestellt werden, besonders geeignet ist ein pH-Wert im Bereich von 2,2. Nach der Phasentrennung kann es zweckmäßig sein, die wässrige Phase noch ein oder mehrmals mit dem betreffenden organischen Lösungsmittel zu extrahieren. Das immobilisierte Enzym kann nach Abschluss der Reaktion bzw. nach der Extraktion wiederverwendet werden.

Alternativ kann als Startmaterial der enzymatischen Umsetzung die Verbindung (II) eingesetzt werden, wobei nach anschließender Verseifung ebenfalls ein Gemisch der Verbindungen der Formel (IV) und (V) oder ein Gemisch von Salzen der Verbindungen der Formel (IV) und (V) erhalten wird, und wobei der pH-Wert zwischen 6,5 und 9,5, bevorzugt 7,5 bis 8,5 ist, und die übrigen Bedingungen den für Verfahrensschritt (B) genannten entsprechen. Im Anschluß an die enzymatische Reaktion der Verbindung (II) wird das entstandene, gewünschte, veresterte Diastereomer verseift, wobei die Bedingungen den für Verfahrensschritt (A) genannten entsprechen. Dieser Verfahrensschritt wird im folgenden zusammenfassend (BA') genannt. Ein weiterer Gegenstand der vorliegenden Erfindung ist daher ein Verfahren zur Herstellung der Verbindung (I) ausgehend von der Verbindung (II) umfassend die Teilschritte (BA'), (C) und entweder (D-A) oder alternativ (D-B.1), (D-B.2.1) oder (D-B.2.2) und (D-B.3) wie oben definiert.

Als wässrige Phase können in Verfahrensschritt (B) bzw. (BA') optional wässrige Puffersysteme mit den entsprechenden, dem Fachmann bekanten pH-Bereichen eingesetzt werden, beispielsweise für einen pH-Wert von 8,0 ein Phosphatpuffer.

Ein weiterer Gegenstand der vorliegenden Erfindung ist ein Gemisch der Verbindungen der Formeln (IV) und (V) 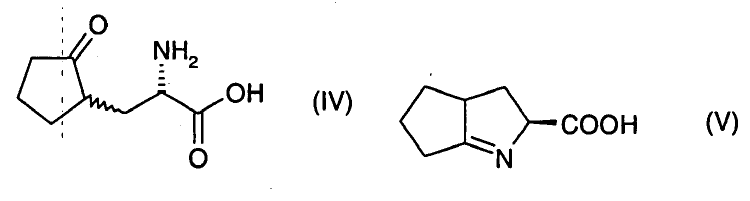 oder ein Gemisch von Salzen der Verbindungen (IV) und (V), vorzugsweise ein Gemisch der Verbindungen (IV)-HCl und (V)-HCl. Das Gemisch der Verbindungen (IV) und (V) kann optional isoliert werden. Vorzugsweise wird das Gemisch der Verbindungen (VI) und (V) in Form eines Säureadditionssalzes isoliert, beispielsweise als Salz von HCl, HBr, H₂SO₄, Methansulfonsäure, Toluensulfonsäure oder Phenylsulfonsäure. Zur Isolierung und zum Ausrühren des in der Aufarbeitung des Gemisches von Salzen der Verbindungen (IV) und (V) erhaltenen Kristallisats eignen sich vorzugsweise wassermischbare Lösungsmittel wie beispielsweise (C₂-C₃)-Alkanole oder Methylethylketon.

Die katalytische Hydrierung in Reaktionsschritt (C) kann nach dem Fachmann bekannten Bedingungen durchgeführt werden. Beispielsweise kann das Verfahren unter den folgenden Bedingungen erfolgen: Die wässrige Phase der Aufarbeitung von Verfahrensschritt (B) oder (BA') enthaltend die Verbindung der Formel (V) oder ein Salz davon im Gemisch mit der Verbindung (IV) oder einem Salz davon wird vorgelegt und ein geeigneter Katalysator wird hinzugefügt. Als Katalysator eignet sich z.B. Platin auf Aktivkohle oder Palladium auf Aktivkohle, besonders geeignet ist Palladium auf Aktivkohle. Bevorzugt ist ein Gewichtsverhältnis von Palladium zu Aktivkohle von 5:95 bis 10:90). Pro Gramm wässriger Lösung wird vorzugsweise eine Katalysatormenge von 1-10 mg, besonders bevorzugt von 3 mg eingesetzt. Dem Reaktionsgemisch kann weitere Aktivkohle hinzugefügt werden, bevorzugt 5 bis 20 Gewichtseinheiten pro Gewichtseinheit Katalysator, besonders bevorzugt 9-13 Gewichtseinheiten. Die Hydrierung wird bei einem Wasserstoffdruck von 5 bis 15 bar, vorzugsweise von 8 bis 12, besonders bevorzugt von ca. 10 bar durchgeführt. Die Reaktionstemperatur kann zwischen 40 °C und der Siedetemperatur des Reaktionsgemisches betragen und liegt vorzugsweise bei 60-100 °C. Eine bevorzugte Reaktionstemperatur liegt bei ca. 80 °C.

Durch die Hydrierung der Verbindung (V) oder einem Salz davon aus dem Gemisch der Verbindungen (IV) und (V) wird das Gleichgewicht zwischen den Verbindungen (IV) und (V) zugunsten der Verbindung (V) verschoben, da ausschließlich die Verbindung (V) hydriert werden kann. Die Verbindung (VI) kann optional isoliert werden. Die Verbindung (VI) kann in freier Form oder in Form eines Säureadditionssalzes isoliert werden, beispielsweise als Salz von HCl, HBr, H₂SO₄, Methansulfonsäure, Toluensulfonsäure oder Phenylsulfonsäure. Zur Isolierung und zum Ausrühren des in der Aufarbeitung der Verbindung (VI) oder Salzen der Verbindung (VI) eignen sich vorzugsweise wassermischbare Lösungsmittel wie beispielsweise (C₂-C₃)-Alkanole oder Methylethylketon.

Die Verbindung (VI) kann vor dem folgenden Schritt (D-A) oder (D-B.1) durch Zugabe von Base, beispielsweise NaOH oder KOH, aus dem Säureadditionssalz freigesetzt werden und anschließend optional durch Elektrodialyse entsalzt werden.

Nach der enzymatischen Racematspaltung in Verfahrensschritt (B) oder (BA') liegt die Verbindung (IV) mit der Stereochemie (2S,3RS) vor. Bei der Hydrierung wird über das vorgelagerte Gleichgewicht der cyclischen Form (V) mit der offenkettigen Form (IV) und die mögliche Racemisierung an Position 3 (wegen einer Keto-Enol-Tautomerie im Sauren) gewährleistet, dass sich ausschließlich die Verbindung (VI) bildet, nicht aber das Diastereomer davon. Das ungewünschte (3R)-Isomer der Verbindung (V) kann wegen der sterischen Hinderung an der Katalysatoroberfläche nur sehr langsam hydriert werden, so dass es über die offenkettige Verbindung (3R)-(IV), mit der es im Gleichgewicht steht, in das (3S)-(IV)-Isomer re-isomerisiert, welches dann weiter zum all-S-Bicyclus (VI) hydriert wird.

Verfahrensschritt (D-A) kann nach dem Fachmann bekannten Bedingungen durchgeführt werden, beispielsweise wie im US-Patent 4,496,541, Beispiel 1, beschrieben. Die Verbindung (VI) wird in einem geeigneten Lösungsmittel, beispielsweise Dichlormethan oder Wasser bei einem pH-Wert von 8-12, vorzugsweise 10-11, mit der Verbindung (VII) vermischt und bei beispielsweise 10-40 °C, vorzugsweise 20-25 °C gerührt.

Die Veresterung der Verbindung (VI) mit Benzylalkohol in Verfahrensschritt (D-B.1) kann nach dem Fachmann bekannten Methoden durchgeführt werden, beispielsweise wie in der europäischen Patentanmeldung EP 79022 A2, Beispiel I (3) beschrieben: Verbindung (VI) wird optional aktiviert, indem sie zunächst mit Methansulfonsäure oder Thionylchlorid in einem geeigneten Lösungsmittel, beispielsweise n-Hexan, n-Heptan, Toluen oder einem Gemisch davon umgesetzt wird; anschließend wird die optional aktivierte Verbindung (VI) mit Benzylalkohol versetzt, wobei die Reaktion vorzugsweise unter Rückfluß durchgeführt wird. Die so erhaltene Verbindung (VIII), Benzyl-(2S, 3aS, 6aS)octahydrocyclopenta[b]pyrrol-2-carboxylat, kann optional isoliert werden. Vorzugsweise wird die Verbindung (VIII) in Form eines Säureadditionssalzes isoliert, beispielsweise als Salze von HCl, HBr, H₂SO₄, Oxalsäure, Phosphorsäure, Methansulfonsäure, Toluensulfonsäure oder Phenylsulfonsäure. Die Verbindung (VIII) wird vorzugsweise vor dem folgenden Schritt (D-B.2.1) oder (D-B.2.2) durch Zugabe von Base, beispielsweise NaOH oder KOH, aus dem Säureadditionssalz freigesetzt.

Verfahrensschritt (D-B.2.1) kann durchgeführt werden, indem Verbindung (VIII) in einem geeigneten Lösungsmittel mit Verbindung (VII) versetzt wird. Geeignete Lösungsmittel sind generell aprotische und nicht-wassermischbare Lösungsmittel, beispielsweise Butylacetat, Ethylacetat, Dichlormethan und Toluol. Die Temperatur der Umsetzung von (VIII) mit (VII) beträgt 5-30 °C, vorzugsweise 10-15 °C. Zur Aufarbeitung kann das Reaktionsgemisch mit einer wässrigen Base, vorzugsweise Natronlauge oder Kalilauge von einem pH-Wert von 10-13, versetzt werden, um überschüssige Verbindung (IX), die durch Hydrolyse aus (VII) entsteht, wässrig zu extrahieren. Die entstandene Verbindung (X) kann optional isoliert werden.

Die Amidbildung in Verfahrensschritt (D-B.2.2) kann nach dem Fachmann bekannten Methoden durchgeführt werden, beispielsweise wie in der europäischen Patentanmeldung EP 79022 A2, Beispiel I (4) beschrieben: Die Verbindung der Formel (VIII) wird in einem geeigneten inerten Lösungsmittel, beispielsweise Ethylacetat, Butylacetat, Dichlormethan oder Dimethylformamid, mit der Verbindung der Formel (IX) bei einer Temperatur von 5-20 °C, vorzugsweise 10-15 °C, in Gegenwart von einem oder mehreren Standard-Amidkupplungsreagenzien, beispielsweise Dicyclohexylcarbodiimid, HOBt, Propanphosphonsäureanhydrid oder Methanphosphonsäureanhydrid gekuppelt, wobei der pH-Wert vorzugsweise zwischen 8 und 9 gehalten wird, beispielsweise mittels Natronlauge. Die so erhaltene Verbindung (X), Benzyl(2S, 3aS, 6aS)-1-2-[[(1S)-1-(Ethoxycarbonyl)-3-phenylpropyl]amino]-(2S)-propanoyl)octahydrocyclopenta[b]pyrrol-2-carboxylat, wird optional isoliert.

Die katalytische Hydrierung in Verfahrensschritt (D-B.3) kann nach dem Fachmann bekannten Methoden durchgeführt werden, beispielsweise wie in der europäischen Patentanmeldung EP 79022 A2, Beispiel I (5) beschrieben: Der Benzylester der Formel (X) wird beispielsweise durch katalytische Hydrierung gespalten, wobei die Hydrierung vorzugsweise in einem geeigneten Lösungsmittel, z.B. einem (C₁-C₃)Alkanol, vorzugsweise Methanol oder Ethanol, bei einer Temperatur von 0-20 °C, bevorzugt 5-10 °C und einem Druck von 0,5 - 3 bar, bevorzugt bei 1,0-2,0 bar unter Zugabe eines geeigneten Katalysators, z.B. Pd/C (10 Gew.-%), katalytisch hydriert wird.

Die Verbindung der Formel (I) kann im Anschluss an die Verfahrensschritte (D-A) oder (D-B.3) mittels Standardmethoden optional weiter aufgereinigt werden, beispielsweise durch chromatographische Methoden oder durch Umkristallisation aus einem geeigneten Lösungsmittel. Ein geeignetes Lösungsmittel ist beispielsweise ein Gemisch aus Methanol und Diisopropylether, oder alternativ Aceton, oder Ethylacetat.

Durch die nachfolgendes Ausführungsbeispiele soll die vorliegende Erfindung erläutert aber nicht beschränkt werden.

### Beispiel 1 - Herstellung der Verbindung der Formel (II) mit R gleich Methyl, (3-(2-Oxo-cyclopentyl)-2-phenylacetylamino-propionsäuremethylester), ausgehend von 3-Chlor-N-phenylacetylalaninmethylester

16 g 3-Chlor-N-phenylacetylalaninmethylester wurden in 100 ml Ethylacetat suspendiert. Bei 20-25°C wurden 20 ml Triethylamin zugegeben und gleich im Anschluss 18 g 1-Cyclopent-1-enyl-pyrrolidin bei 20-30°C innerhalb 20 Minuten zugetropft. Es wurde auf 40-45°C erhitzt und anschließend 2 Stunden bei dieser Temperatur nachgerührt. Es wurden 20 ml Wasser zugegeben und mit ca. 20 ml Salzsäure (30 %) bei 10-13 °C auf pH zwischen 1 und 3 eingestellt. Es wurde 5 Minuten nachgerührt und anschließend die Phasen getrennt. Die organische Phase wurde mit 10 ml Wasser extrahiert. Die organische Phase wurde bei bis zu einem Restvolumen von ca. 50 ml im Vakuum eindestilliert. Es wurde auf 0-5 °C abgekühlt, dann mit ca. 0,1-0,2 g 3-(2-Oxo-cyclopentyl)-2-phenylacetylamino-propionsäuremethyl ester angeimpft. Es bildete sich nach ca. 10 Minuten eine Suspension, dann wurde 20 Minuten nachgerührt. Anschließend wurden 45 g Diisopropylether zugegeben. Es bildete sich eine gut rührbare Suspension. Es wurde 30 Minuten bei 0-5 °C nachgerührt, dann abgesaugt, mit 2 x 20 ml Diisopropylether (vorgekühlt auf 5-10 °C) gewaschen und im Vakuumtrockenschrank bei 50°C getrocknet. Es wurden 15,95 g (86,8 %) Produkt erhalten.

### Beispiel 2 - Herstellung der Verbindung der Formel (II) mit R gleich Methyl ausgehend von N-Phenylacetylserinmethylester

In einen Kessel wurden 20,0 kg N-Phenylacetylserinmethylester eingetragen. Anschließend wurden in den Kessel 120,0 L Ethylacetat vorgelegt. Die Suspension wurde unter Rühren auf 40-45 °C erwärmt. Anschließend wurde in den Kessel ein Gemisch aus 4,0 kg Phosphortrichlorid und 6,0 L Ethylacetat aus einer Vorlage bei 40-45 °C innerhalb von 30 - 45 Minuten zugegeben und mit 6,0 L Ethylacetat in den Kessel nachgewaschen. Das Reaktionsgemisch wurde bei 40-45 °C für 45-60 Minuten nachgerührt. Bei einer maximalen Manteltemperatur von 45 °C wurden aus dem Reaktionsgemisch unter Vakuum 20-25 L Ethylacetat abdestilliert, wobei der Überschuß an Phophortrichlorid entfernt wurde. Das Reaktionsgemisch wurde auf 20-30 °C abgekühlt, dann wurden 15,0 L Ethylacetat zugegeben. In den Reaktionskessel wurden dann bei 20-30 °C innerhalb von 15-30 Minuten 18,9 kg Triethylamin zugegeben und mit 5,0 L Ethylacetat nachgewaschen. Anschließend wurden in den Kessel 20,1 kg 1-Cyclopent-1-enyl-pyrrolidin innerhalb von 20-30 Minuten bei 20-30 °C zugegeben. Es wurde mit 5,0 L Ethylacetat nachgewaschen. Das Reaktionsgemisch wurde bei 20-30 °C für mindestens 3 Stunden nachgerührt. Zur Aufarbeitung wurden 25 L Wasser zugegeben. Das Reaktionsgemisch wurde auf 10-15 °C temperiert. Anschließend würde der pH-Wert des Reaktionsgemisches durch Zugabe von 30 % Salzsäure auf 2,0-2,5 eingestellt. Das Reaktionsgemisch wurde 10-20 Minuten nachgerührt. Dann wurden die Phasen getrennt und die Ethylacetatphase bei 20-25 °C mit 15 L Wasser versetzt und 10-20 Minuten nachgerührt. Die Phasen wurden erneut getrennt. Die vereinigten Wasserphasen wurden bei 20-25 °C mit 15 L Ethylacetat versetzt und 10-20 Minuten extrahiert. Die organische Phase wird unter Vakuum bei einer max. 70 °C auf ein Restvolumen von 18-23 L eingeengt. Der Destillationsrückstand wurde auf -3 °C bis +3 °C abgekühlt und dann mit 20 g 3-(2-Oxo-cyclopentyl)-2-phenylacetylamino-propionsäuremethyl ester angeimpft. Es wurde 60-120 Minuten bei -3 °C bis +3 °C nachgerührt. Dann wurde die Produktsuspension mit 80,0 L Diisopropylether versetzt und nochmals 60-90 Minuten bei -3 °C bis +3 °C nachgerührt. Die Suspension wurde auf einem Drucktrichter isoliert. Das Isolat wurde zweimal mit jeweils 10,0 L Diisopropylether gewaschen. Das Material wurde bei 30-40 °C getrocknet. Es wurden 13,7 kg Produkt (Reinheit 95,7 %, Ausbeute 51,2 %) erhalten.

### Beispiel 3 - Verseifung der Verbindung der Formel (II) mit R gleich Methyl zur Verbindung der Formel (III) [Schritt (A)]

In einen Kessel wurden 10,8 kg Verbindung (II) mit R = CH₃, 3-(2-Oxo-cyclopentyl)-2-phenylacetylamino-propionsäuremethylester, 23,7 L Wasser und 5,5 kg Natronlauge . (33 %) vorgelegt. Das Reaktionsgemisch wurde auf 45 °C erwärmt und bei dieser Temperatur 5 Stunden gerührt. Es wurde auf 20 °C abgekühlt und der pH-Wert bei dieser Temperatur mit Salzsäure (30 %) auf pH 6,4 eingestellt. Das Reaktionsgemisch wurde mit 30 L Wasser verdünnt. Die in der Reaktion entstehende, als Gemisch von 2 diastereomeren Racematen vorliegende Verbindung 3-(2-Oxo-cyclopentyl)-2-phenylacetylamino-propionsäure (III) wurde im folgenden Schritt ohne weitere Aufreinigung als wässrige Lösung weiterverarbeitet.

### Beispiel 4 - Enzymatische Racematspaltung mittels Penicillin-G-amidase (Herstellung der Verbindung der Formel (V)) [Schritt (B)]

Zum Reaktionsgemisch aus Beispiel 3 enthaltend [2RS, 3RS]-3-(2-Oxo-cyclopentyl)-2-phenylacetylamino-propionsäure der Formel (III) wurden 2,5 kg polymergebundene Penicillin-G-amidase (PGA450, Firma Roche Diagnostics GmbH, Mannheim, Deutschland, Bestellnummer 1414208) zugegeben. Es wurde mit 7,7 L Wasser nachgespült. Das Reaktionsgemisch wurde auf 28 °C erwärmt und bei dieser Temperatur 5 Stunden bei einem pH-Wert von 6,4 nachgerührt. Das Reaktionsgemisch wurde anschließend filtriert.

Zum Reaktionsgemisch wurden 16,2 L Ethylacetat zugegeben, und der pH-Wert des Gemisches wurde mit Salzsäure (30 %) auf pH 2,2 eingestellt. Nach Phasentrennung wurde die wässrige Phase noch dreimal mit jeweils 16,2 L Ethylacetat extrahiert. Die wässrige Phase enthaltend das gewünschte Produkt (2S,3RS)-2-Amino-3-(2-oxo-cyclopentyl)-propionsäure-hydrochlorid (IV)-HCl, das unter Wasserabspaltung im Gleichgewicht mit 2,3,3a,4,5,6-Hexahydro-cyclopenta[b]pyrrol-2-carbonsäurehydrochlorid (V)-HCl steht, wurde ohne weitere Aufarbeitung in der folgenden Reaktion umgesetzt.

### Beispiel 5 - Umsetzung der Verbindung (II) mit Penicillin-G-Amidase [Schritt (BA')]

Verbindung (II) wurde in einem Gemisch von iso-Propanol und Wasser (50 % w/v) gelöst und 0.2 M Kaliumphosphatbuffer pH 8.0 zugegeben, bis eine Endkonzentration von (II) von 60 g/L einstellt war. Die Reaktionsmischung wurde mit 0.8 kU pro g der Verbindung (II) polymergebundene Penicillin-G-amidase (PGA450, Firma Roche Diagnostics GmbH, Mannheim, Deutschland, Bestellnummer 1414208) versetzt und bei 28° C inkubiert. Die Reaktion wurde nach 24 Stunden beendet, indem 85%ige Phosphorsäure bis zu einem pH-Wert von 2 zugegeben wurde. Die unerwünschten Enantiomeren wurden durch Extraktion mit Essigsäureethylester extrahiert, das gewünschte Produkt befand sich in der wässrigen Phase.

In analoger Weise können die Reaktionen aus Beispiel 4 und wie hier vorstehend in Beispiel 5 mit den Penicillin-G-Amidasen von der Firma Fermenta Biotech Ltd. oder der Firma Fluka oder mit anderen geeigneten Penicillin-G-Amidasen durchgeführt werden.

### Beispiel 6 - Isolierung des Gemisches der Verbindungen (IV)-HCl und (V)-HCl

400 g einer Lösung eines Gemisches von (IV)-HCl und (V)-HCl laut Beispiel 4 wurden bei 50°C und einem Druck von 30-50 mbar bis zu einem öligen Rückstand eindestilliert, der beim Stehen kristallisiert. Das Kristallisat wurde bei 50°C mit 700 ml Aceton 1 Stunde verrührt, auf 0-3 °C abgekühlt, 1 Stunde nachgerührt, abgesaugt und mit 100 ml Aceton gewaschen. Das Produkt wurde bei 30 °C im Vakuum getrocknet. Das Gemisch der Verbindungen (IV)-HCl und (V)-HCl wurde als hellbraunes Pulver erhalten, Auswage: 46,9 g hellbraunes Pulver.

### Beispiel 7 - Hydrierung der Verbindung (V) zur Verbindung der Formel (VI) [Schritt (C)]

Die wässrige Phase der Aufarbeitung aus Beispiel 4 wurde unter Zusatz von 140 g Palladium (5 % auf Aktivkohle) und 540 g Aktivkohle versetzt. Es wurde ein Druck von 10 bar angewendet. Dann wurde die Temperatur auf 80 °C erhöht. Bei dieser Temperatur wurde das Reaktionsgemisch 5 Stunden hydriert. Zur Aufarbeitung wurde das Reaktionsgemisch filtriert. Die wässrige Phase wurde mit Salzsäure auf pH 2 eingestellt und etwaige Nebenprodukte durch Extraktion mit entfernt. Die wässrige Phase enthielt Verbindung (VI), (2S, 3aS, 6aS)Octahydrocyclopenta[b]pyrrol-2-carbonsäure, als, Hydrochlorid.

### Beispiel 8 - Isolierung der Verbindung (VI)

206 g einer durch Elektrodialyse entsalzten Lösung der Verbindung (VI) wurden am Rotationsverdampfer bei 50 °C im Vakuum innerhalb 2 Stunden auf 110 g Restgewicht eindestilliert. Zu der Suspension wurden 500 ml Aceton gegeben und auf 0-3 °C abgekühlt und 2 Stunden nachgerührt. Nach Absaugen wurde mit 50 ml kaltem Aceton gewaschen und bei 50 °C im Vakuum getrocknet. Man erhielt 32,6 g Verbindung (VI) als weißliches Pulver.

### Beispiel 9 - Benzylierung des Hydrochlorids der Verbindung (VI) zum Hydrochlorid der Verbindung (VIII) [Schritt (D-B.1)]

Die wässrige Phase der Aufarbeitung der in Beispiel 7 beschriebenen Hydrierung enthaltend 1,76 kg der Verbindung der Formel (VI) wurden mit 56,5 g 33%iger NaOH auf pH 6,3 gestellt. Diese Lösung wurde filtriert, mit wenig Wasser gewaschen und in eine Elektrodialyseanlage überführt. Die Lösung wurde bei 14 V, 180-200 L/h Umlaufgeschwindigkeit 62 Minuten dialysiert, bis der Stromstärkewert nicht mehr fiel.

Die wässrige entsalzte Lösung der Verbindung (VI) wurde am Rotationsverdampfer bei 40-50°C im Vakuum auf 140g Restmasse eingeengt. Diese Lösung wurde bei 20 bis maximal 30°C langsam unter Eiskühlung mit 45 g Methansulfonsäure versetzt und 30 Minuten nachgerührt.

Die wässrige (VI)-Mesylat Lösung wurde am Rotationsverdampfer bei 40-50 °C im Vakuum auf 140 g Restmasse eingeengt. Zu dieser Lösung wurden 140 ml n-Heptan zugegeben. Unter Rühren wurden 39 g Benzylalkohol zugesetzt. Das Reaktionsgemisch wurde unter Normaldruck zum Rückfluss erhitzt und so lange Wasser ausgekreist, bis die Auskreisgeschwindigkeit etwa 0,3 g/Stunde liegt. Anschließend wurden unter Wasserabscheidung 40,8 g Benzylalkohol innerhalb von 40 Minuten zudosiert. Es wurde 2 Stunden unter Rückfluss nachgerührt bis kein Wasser mehr abgeschieden wurde. Das Reaktionsgemisch wurde auf etwa 10°C abgekühlt und 187 ml Wasser zugetropft. Die Phasen wurden getrennt. Die obere, organische Phase wurde erneut mit 10 ml Wasser extrahiert. Die vereinigten Wasserphasen wurden mit 136 g Ethylacetat versetzt und auf 0-10°C gekühlt. Anschließend wurde mit 25 ml NaOH 33%ig ein pH von 10-10,5 eingestellt. Es wurde 15 Minuten bei 0-.10°C gerührt und anschließend die Phasen getrennt. Die organische Phase wurde mit 15 ml Wasser gewaschen. Die vereinigten Wasserphasen wurden mit 20 ml Ethylacetat gewaschen. Die organischen Phasen enthaltend die Verbindung (VIII) wurden vereinigt und mit Ethylacetat auf 250 g verdünnt. Mit 23 ml HCl 30%ig wurde bei 5-10°C ein pH-Wert von 1 eingestellt, wobei die Verbindung (VIII), Benzyl-(2S, 3aS, 6aS)octahydrocyclopenta[b]pyrrol-2-carboxylat, als Hydrochlorid kristallisierte. Es wurde 1 Stunde bei 0-5°C nachgerührt, das Produkt abgesaugt und mit 50 ml kaltem Ethylacetat gewaschen. Das Produkt wurde bei 40°C im Vakuum getrocknet.

### Beispiel 10 - Herstellung der Verbindung (I) [Schritte (D-B.2.1) und (D-B.3)]

10,0 g (VIII)-Hydrochlorid aus Beispiel 9 wurden zusammen mit 40 ml Butylacetat und 87 ml Wasser in seinem 250 ml-Kolben vorgelegt. Bei 20-25 °C wurde unter Rühren mit 33%iger NaOH der pH-Wert auf 10,5 eingestellt und gehalten. Es wurde 30 Minuten gerührt bis der pH-Wert nicht mehr fällt. Die Phasen wurden getrennt und die wässrige Phase mit 5 ml Butylacetat nachextrahiert. Die organischen Phasen wurden vereinigt und am Rotationsverdampfer bei 50°C im Vakuum vollständig bis zur Gewichtskonstariz eingeengt.

Die als Öl verbleibende Verbindung (VIII) wurde mit 45ml Butylacetat aufgenommen und 10,6 g (2S)-2-[(4S)-4-Methyl-2,5-dioxo-oxazolidin-3-yl]-4-phenylbuttersäureethylester (VII) bei 20-25°C innerhalb von 30 Minuten eingetragen. Das Reaktionsgemisch wurde 60 Minuten nachgerührt. Es wurden 25ml Wasser zugegeben, 10 Minuten nachgerührt und die Phasen getrennt. Die organische Phase wurde am Rotationsverdampfer bei 50°C im Vakuum vollständig bis zur Gewichtskonstanz eingeengt. Die verbliebene ölige Verbindung (X), (2S, 3aS, 6aS)-1-[2-[(1S)-1-Ethoxycarbonyl-3-phenyl-propylamino]-(2S)-propionyl]-octahydro-cyclopenta[b]pyrrol-2-carbonsäure-benzylester, wurde mit 200 ml Methanol aufgenommen. Es wurden 0,7 g Pd/C (5 Gewichts-%) feucht zugegeben und das Reaktionsgemisch bei 10 °C bei 3 bar H₂-Druck 1,5 Stunden hydriert. Nach Abfiltrieren des Katalysators wurde das Filtrat am Rotationsverdampfer bei 20 °C Badtemperatur im Vakuum auf 25 g eingeengt und mit 75 ml Diisopropylether versetzt. Es wurde auf 0-3 °C abgekühlt, 1 Stunde nachgerührt, das kristallisierte Produkt abgesaugt und zweimal mit jeweils 15 ml kaltem Diisopropylether gewaschen. Die so erhaltene Verbindung (I) wurde bei < 30 °C im Vakuum getrocknet.

### Beispiel 11 - Herstellung der Verbindung (X)

9,62 g der Verbindung (VIII) wurden in 20 ml Butylacetat gelöst. Bei 10-15 °C wurden innerhalb von 30 Minuten 89,76 g der Verbindung (VII) als 13,9%ige (1,06 M) Lösung von Verbindung (VII) zugetropft. Es wurde 60 Minuten nachgerührt. Zur Aufarbeitung wurden 30 ml Wasser zugegeben und mit 33,2 ml NaOH (11%ig) auf pH 11 gestellt und 30 Minuten nachgerührt. Nach Phasentrennung wurde die organische Phase am Rotationsverdampfer bei 50°C im Vakuum vollständig bis zur Gewichtskonstanz eingeengt. 19,0 g der Verbindung (X) wurden als zähes Öl isoliert.

### Beispiel 12 - Herstellung der Verbindung (I) [Schritt (D-A)]

Das Reaktionsgemisch aus der Hydrierung der Verbindung (V) enthaltend (VI)-HCl (Beispiel 7) wurde nach Filtration des Hydrierkatalysators bei 20-25 °C mit Natronlauge (33 %) auf pH 10,0-11,0 eingestellt. Dann wurden 5,1 kg 2-(4-Methyl-2,5-dioxo-oxazolidin-3-yl)-4-phenyl-buttersäureethylester (VII) eingetragen. Das Gemisch wurde bei 20-25 °C 3-4 Stunden nachgerührt. Dabei wurde der pH-Wert bei 10,0-11,0 durch Zugabe von Natronlauge konstant gehalten. Zu dem Reaktionsgemisch wurden 2,7 L Aceton zugegeben. Dann wurde der pH-Wert bei 15-20 °C mit Salzsäure (30 %) auf 5,0-5,2 eingestellt. Es wurde mit 50 g Ramipril (I) angeimpft und 30-45 Minuten nachgerührt. Der pH-Wert wurde mit Salzsäure (30 %) auf einem pH-Wert von 4,4-4,6 eingestellt und mindestens 2 Stunden bei 15-20 °C nachgerührt. Das Produkt wurde auf einem Druckfilter isoliert. Der Filterkuchen wurde mit 11 L Wasser nachgewaschen. Wasserfeuchtes Ramipril (roh) wurde in einem Trockenschrank bei < 30 °C getrocknet. Es wurde auf einen Wassergehalt von weniger als 5 % getrocknet.

### Beispiel 13 - Aufreinigung der Verbindung (I) durch Umkristallisation

Es wurden 5,42 kg Verbindung (I) aus Beispiel 12 mit einem Wassergehalt von weniger als 5 % wurden vorgelegt. Anschließend wurden 10,0 L Methanol zugegeben. Das Gemisch wurde unter Rühren auf 25-28 °C erwärmt und bei dieser Temperatur 60-120 Minuten gerührt. Dann wurde der Kesselinhalt über einen Druckfilter filtriert und mit 5,0 L Methanol nachgewaschen. Die Lösung wurde bei einer Manteltemperatur von max. 30 °C im Vakuum eingeengt, wobei ca. 8-10 L Methanol abdestilliert wurden. Anschließend 27,1 L Diisopropylether zum Fällen des Produktes zugegeben. Die Suspension wurde auf 0-5 °C abgekühlt und mindestens 3 Stunden bei dieser Temperatur nachgerührt Die Suspension wurde auf einem Druckfilter isoliert. Das Produkt wurde zweimal mit jeweils 5,4 L Diisopropylether von 20-25 °C gewaschen und anschließend trockengeblasen. Die so aufgereinigte, feuchte Verbindung (I) wurde in einem Trockenschrank bei < 30 °C getrocknet.

Durch die nachfolgenden Patentsprüche, die auch Gegenstand der Beschreibung sein sollen, wird die vorliegenden Erfindung näher erläutert.

## Patentansprüche

1. Verfahren zur Herstellung von Ramipril der Formel (I) 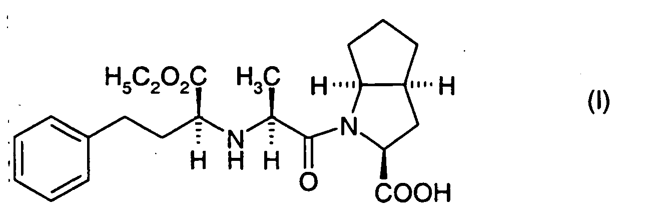 **dadurch gekennzeichnet, dass**
(A) eine Verbindung der Formel (II) 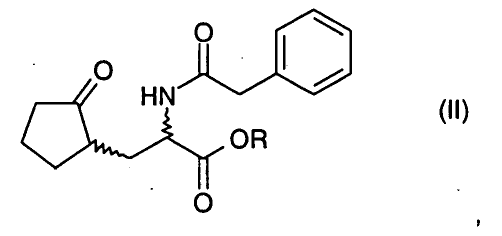 in der R (C₁-C₄)-Alkyl bedeutet, in einem geeigneten Lösungsmittel unter Zusatz einer oder mehreren Basen oder Säuren zu einer Verbindung der Formel (III) 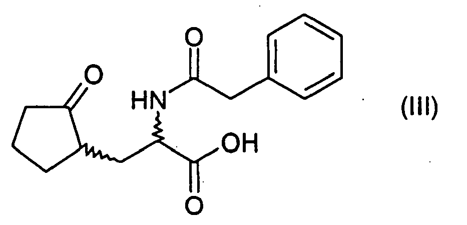 verseift wird, und anschließen
(B) die Verbindung der Formel (III) unter Zugabe von Penicillin-G-Amidase zu einem Gemisch der Verbindungen der Formeln (IV) und (V) 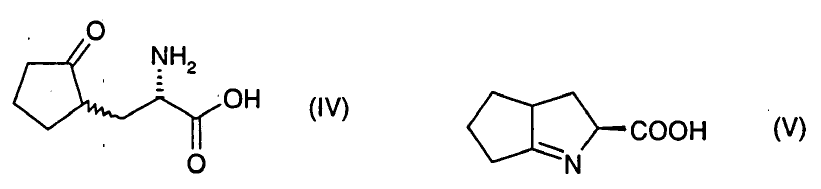 oder einem Gemisch von Salzen der Verbindungen der Formel (IV) und (V) umgesetzt wird,
oder alternativ
(BA') die Verbindung der Formel (II) mit Penicillin-G-Amidase umgesetzt und anschließend unter Zusatz von einer oder mehreren Basen oder Säuren zu einem Gemisch der Verbindungen der Formeln (IV) und (V) oder einem Gemisch von Salzen der Verbindungen der Formel (IV) und (V) verseift wird,
und anschließend
(C) die Verbindung (V) oder ein Salz der Verbindung (V) aus dem Gemisch der Verbindungen (IV) und (V) oder deren Salzen durch katalytische Hydrierung zu einer Verbindung der Formel (VI) 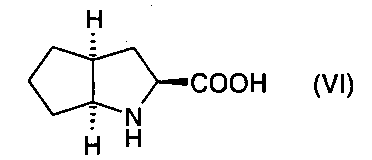 oder einem Salz davon umgesetzt wird, und anschließend entweder
(D-A) die Verbindung der Formel (VI) mit einer Verbindung der Formel (VII) 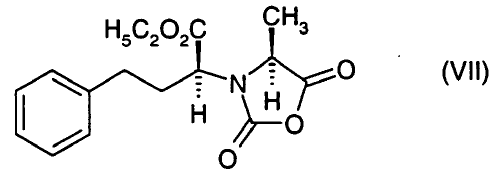 zur Verbindung der Formel (I) umgesetzt wird,
oder alternativ
(D-B.1) die Verbindung der Formel (VI) mit Benzylalkohol zu einer Verbindung der Formel (VIII) 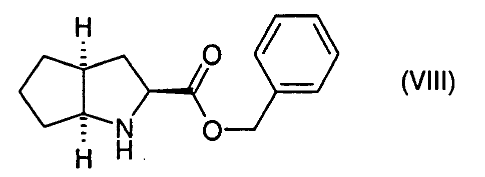 oder einem Salz davon umgesetzt wird, und anschließend entweder
(D-B.2.1) die Verbindung der Formel (VIII) entweder mit der Verbindung der Formel (VII) zu einer Verbindung der Formel (X) 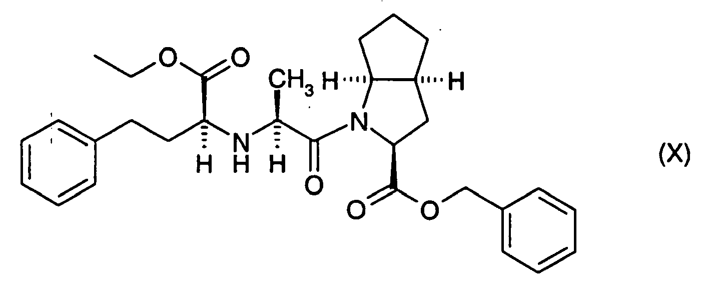 umgesetzt wird, oder alternativ
(D-B.2.2) die Verbindung der Formel (VIII) mit einer Verbindung der Formel (IX) 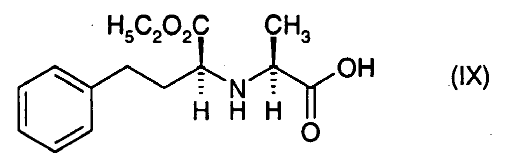 zu einer Verbindung der Formel (X) umgesetzt wird, und anschließend
(D-B.3) aus der Verbindung der Formel (X) durch katalytische Hydrierung die Verbindung der Formel (I) gebildet wird.

2. Verfahren zur Herstellung eines Gemisches der Verbindungen der Formeln (IV) und (V) oder eines Gemisches von Salzen der Verbindungen der Formel (IV) und (V) 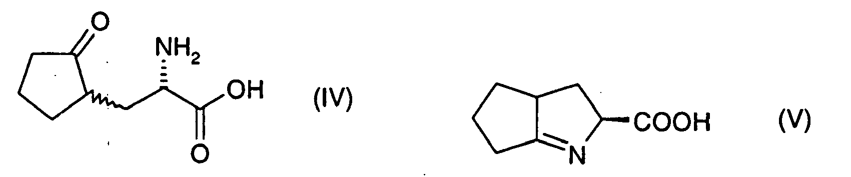 **dadurch gekennzeichnet, dass** eine Verbindung der Formel (II) 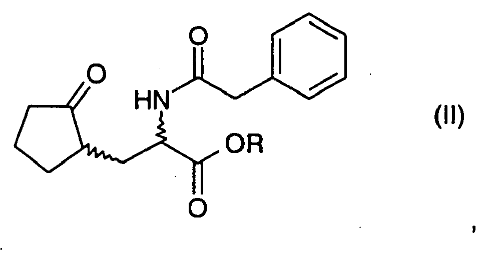 in der R (C₁-C₄)-Alkyl bedeutet, mit Penicillin-G-Amidase behandelt wird, und anschließend mit einer oder mehreren Basen oder Säuren verseift wird.

3. Verfahren zur Herstellung eines Gemisches der Verbindungen der Formeln (IV) und (V) oder eines Gemisches von Salzen der Verbindungen der Formel (IV) und (V) 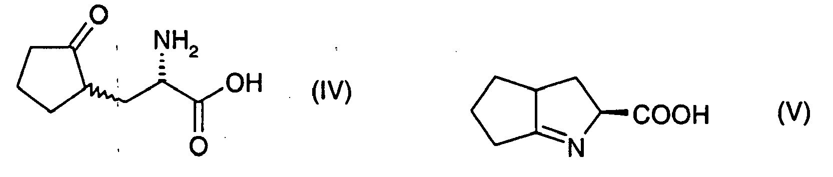 **dadurch gekennzeichnet, dass** eine Verbindung der Formel (III) 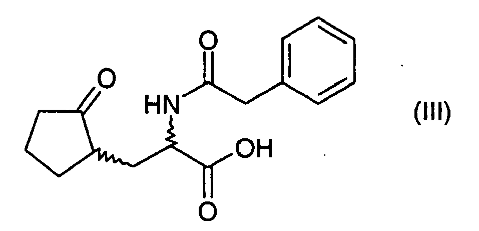 mit Penicillin-G-Amidase behandelt wird.

4. Verbindung der Formel (II) 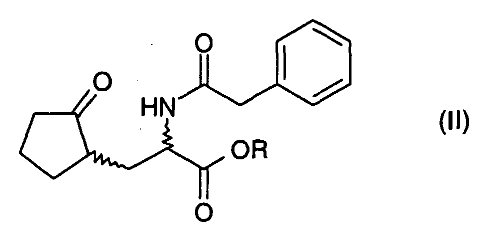 in der R (C₁-C₄)-Alkyl bedeutet.

5. Verwendung einer Verbindung der Formel (II) gemäß Anspruch 4 zur Herstellung von Ramipril.

6. Verbindung der Formel (III)

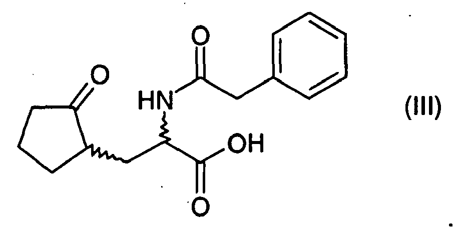

7. Verwendung einer Verbindung der Formel (III) gemäß Anspruch 6 zur Herstellung von Ramipril.

8. Gemisch enthaltend eine isomerenreine Verbindung der Formel (IV) 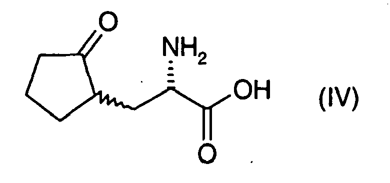 und eine Verbindung der Formel (V) 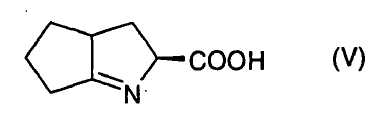 oder enthaltend ein Gemisch von Salzen der Verbindungen der Formel (IV) und (V).

9. Verwendung des Gemisches der Verbindungen der Formeln (IV) und (V) oder eines Gemisches von Salzen der Verbindungen der Formel (IV) und (V) gemäß Anspruch 8 zur Herstellung von Ramipril.

## Claims

1. A method for preparing ramipril of the formula (I) 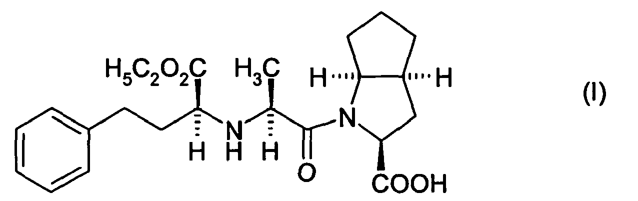 which comprises
(A) hydrolyzing a compound of the formula (II) 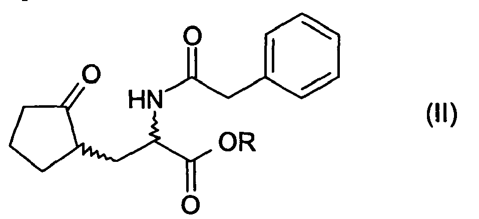 in which R is (C₁-C₄)-alkyl, in a suitable solvent with the addition of one or more bases or acids to a compound of the formula (III) 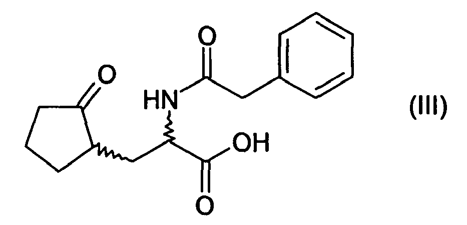 and then
(B) converting the compound of the formula (III) by addition of penicillin G amidase into a mixture of the compounds of the formulae (IV) and (V) 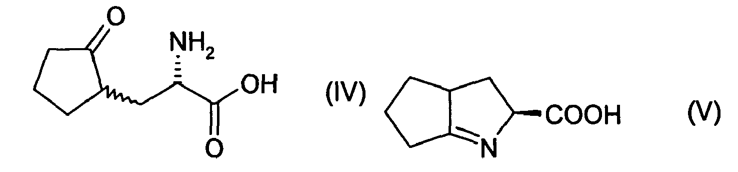 or a mixture of salts of the compounds of the formula (IV) and (V),
or alternatively
(BA') the compound of the formula (II) being reacted with penicillin G amidase and then being hydrolyzed with the addition of one or more bases or acids to a mixture of the compounds of the formulae (IV) and (V) or a mixture of salts of the compounds of the formula (IV) and (V),
and then
(C) converting the compound (V) or a salt of the compound (V) from the mixture of the compounds (IV) and (V) or salts thereof by catalytic hydrogenation into a compound of the formula (VI) 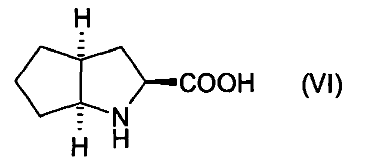 or a salt thereof, and then either
(D-A) reacting the compound of the formula (VI) with a compound of the formula (VII) 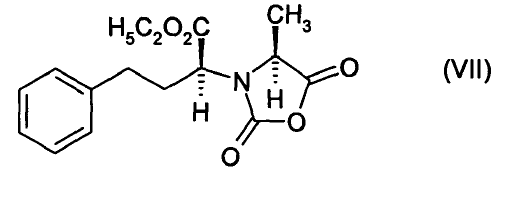 to give the compound of the formula (I),
or alternatively
(D-B.1) reacting the compound of the formula (VI) with benzyl alcohol to give a compound of the formula (VIII) 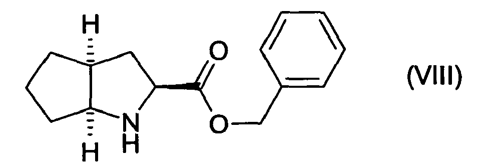 or a salt thereof, and then either
(D-B.2.1) reacting the compound of the formula (VIII) either with the compound of the formula (VII) to give a compound of the formula (X) 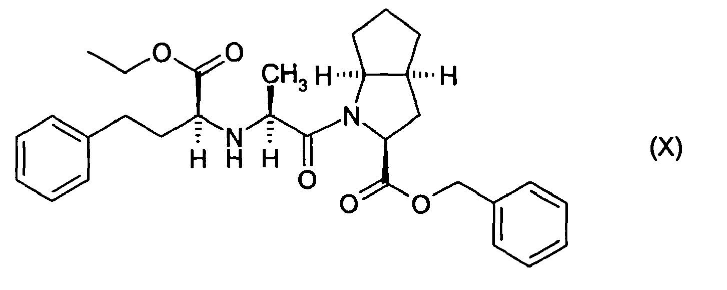 or alternatively
(D-B.2.2) reacting the compound of the formula (VIII) with a compound of the formula (IX) 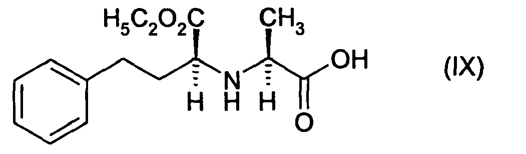 to give a compound of the formula (X), and then
(D-B.3) forming the compound of the formula (I) from the compound of the formula (X) by catalytic hydrogenation.

2. A method for preparing a mixture of the compounds of the formulae (IV) and (V) or a mixture of salts of the compounds of the formula (IV) and (V) 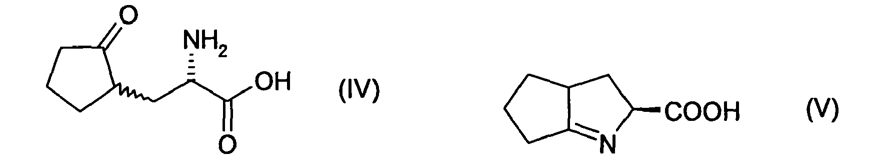 which comprises treating a compound of the formula (II) 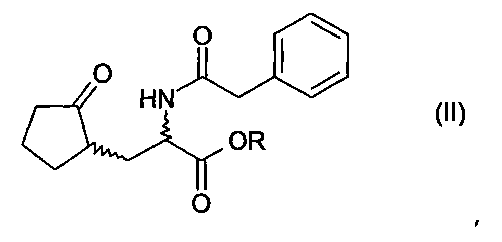 in which R is (C₁-C₄)-alkyl, with penicillin G amidase, and then hydrolyzing with one or more bases or acids.

3. A method for preparing a mixture of the compounds of the formulae (IV) and (V) or a mixture of salts of the compounds of the formula (IV) and (V) 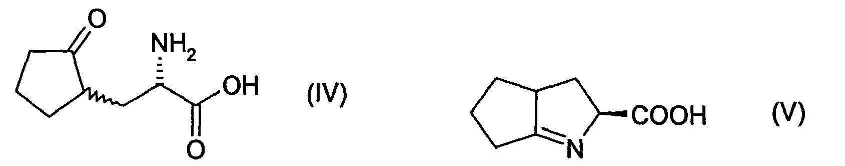 which comprises treating a compound of the formula (III) 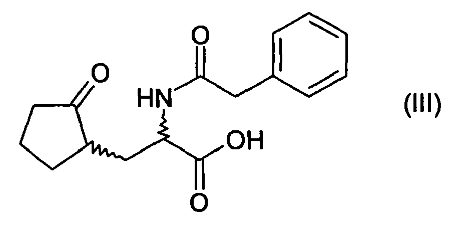 with penicillin G amidase.

4. A compound of the formula (II) 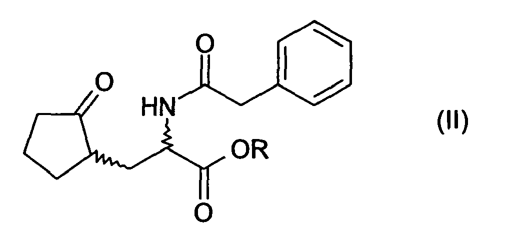 in which R is (C₁-C₄)-alkyl.

5. The use of a compound of the formula (II) as claimed in claim 4 for preparing ramipril.

6. A compound of the formula (III)

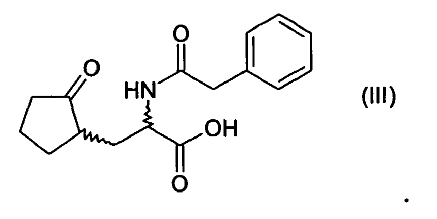

7. The use of a compound of the formula (III) as claimed in claim 6 for preparing ramipril.

8. A mixture comprising an isomerically pure compound of the formula (IV) 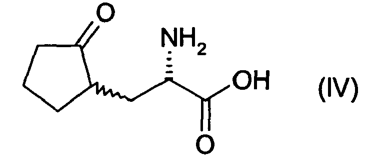 and a compound of the formula (V) 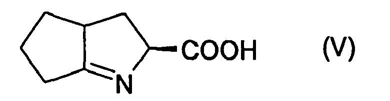 or comprising a mixture of salts of the compounds of the formula (IV) and (V).

9. The use of the mixture of the compounds of the formulae (IV) and (V) or of a mixture of salts of the compounds of the formula (IV) and (V) as claimed in claim 8 for preparing ramipril.

## Revendications

1. Procédé de préparation du ramipril de formule (I) 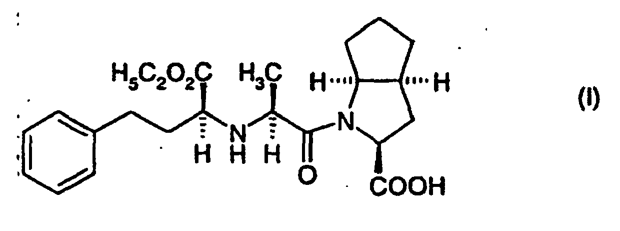 **caractérisé en ce que**
(A) on saponifie un composé de formule (II) 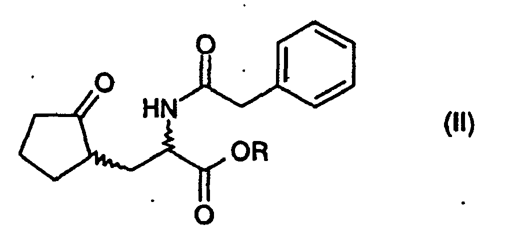 dans laquelle R représente un groupe alkyle en C₁-C₄, dans un solvant approprié, avec addition d'une ou plusieurs bases ou d'un ou plusieurs acides, pour aboutir à un composé de formule (III) 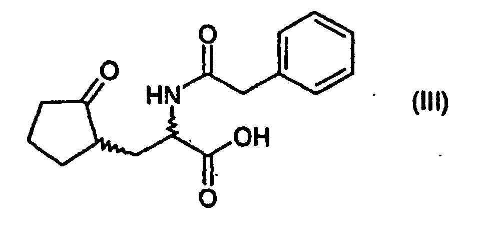 et ensuite
(B) on convertit le composé de formule (III), par addition de pénicilline G amidase, en un mélange des composés des composés de formules (IV) et (V) 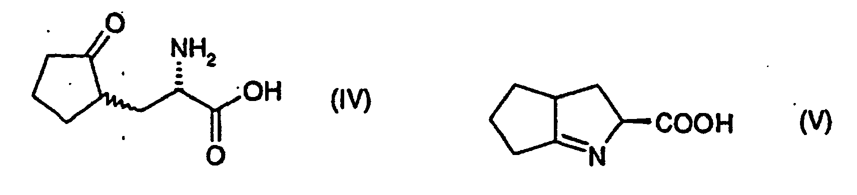 ou en un mélange de sels des composés de formules (IV) et (V),
ou bien
(BA') on fait réagir le composé de formule (II) avec de la pénicilline G amidase et ensuite on saponifie avec addition d'une ou plusieurs bases ou d'un ou plusieurs acides, pour aboutir à un mélange de composés de formules (IV) et (V) ou à un mélange de sels des composés de formules (IV) et (V),
et ensuite
(C) on convertit le composé (V) ou un sel du composé (V) à partir du mélange des composés (IV) et (V) ou de leurs sels, par hydrogénation catalytique, en un composé de formule (VI) 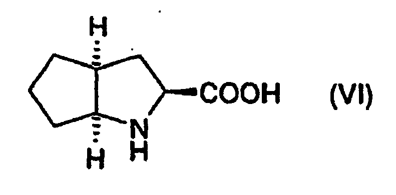 ou en un sel de celui-ci, et ensuite soit
(D-A) on fait réagir le composé de formule (VI) avec un composé de formule (VII) 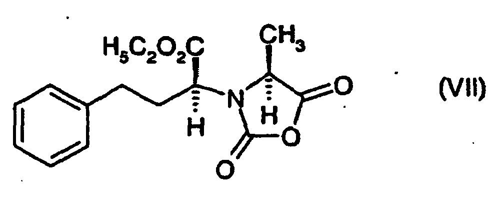 pour aboutir au composé de formule (I),
soit
(D-B.1) on fait réagir le composé de formule (VI) avec de l'alcool benzylique, pour aboutir à un composé de formule (VIII) 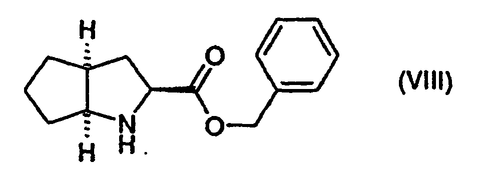 ou à un sel de celui-ci, et ensuite soit
(D-B.2.1) on fait réagir le composé de formule (VIII) soit avec le composé de formule (VII), pour aboutir à un composé de formule (X) 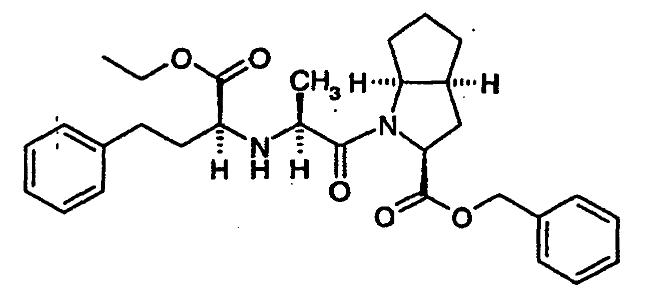 soit
(D-B.2.2) on fait réagir le composé de formule (VIII) avec un composé de formule (IX) 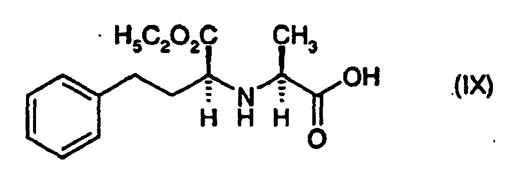 pour aboutir à un composé de formule (X), et ensuite
(D-B.3) le composé de formule (I) est formé par hydrogénation catalytique à partir du composé de formule (X).

2. Procédé pour la préparation d'un mélange des composés de formules (IV) et (V) ou d'un mélange de sels des composés de formules (IV) et (V) 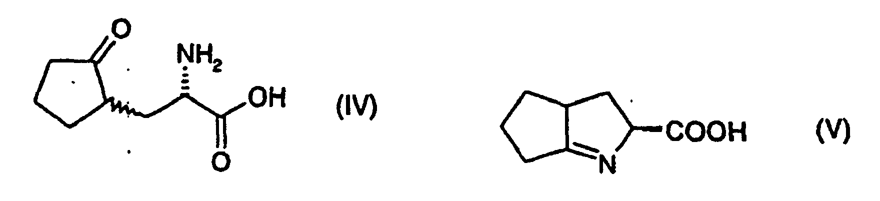 **caractérisé en ce qu'**on traite par de la pénicilline G amidase un composé de formule (II) 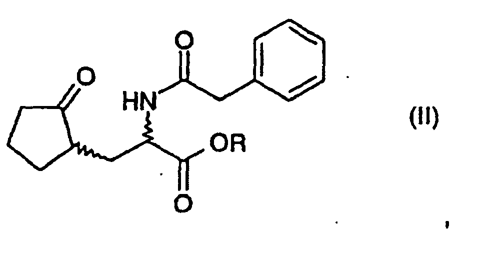 dans laquelle R représente un groupe alkyle en C₁-C₄, et ensuite on saponifie avec une ou plusieurs bases ou un ou plusieurs acides.

3. Procédé pour la préparation d'un mélange des composés de formules (IV) et (V) ou d'un mélange de sels des composés de formules (IV) et (V) 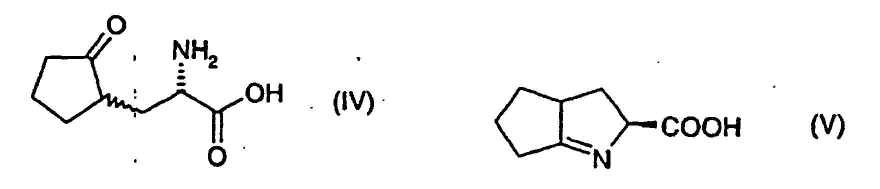 **caractérisé en ce qu'**on traite par de la pénicilline G amidase un composé de formule (III).

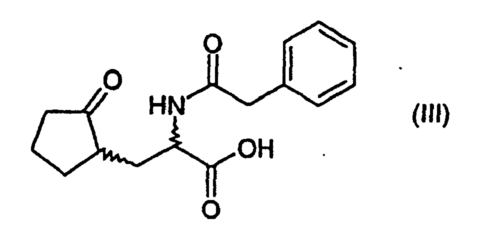

4. Composé de formule (II) 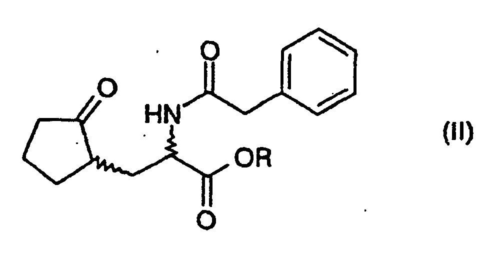 dans laquelle R représente un groupe alkyle en C₁₋C₄.

5. Utilisation d'un composé de formule (II) selon la revendication 4, pour la préparation du ramipril.

6. Composé de formule (III)

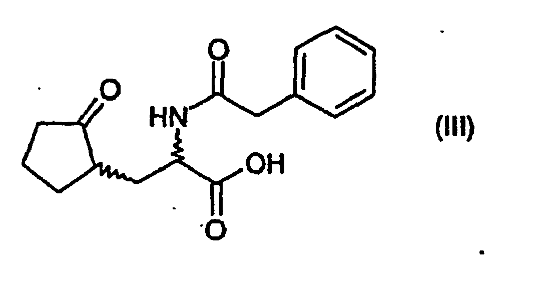

7. Utilisation d'un composé de formule (III) selon la revendication 6, pour la préparation du ramipril.

8. Mélange contenant un composé de formule (IV), sous forme d'isomère pur, 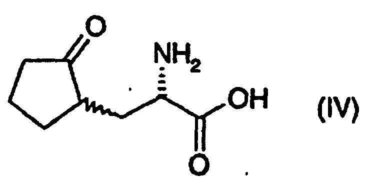 et un composé de formule (V) 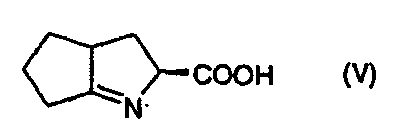 ou contenant un mélange de sels des composés de formules (IV) et (V).

9. Utilisation du mélange des composés de formules (IV) et (V) ou d'un mélange de sels des composés de formules (IV) et (V) selon la revendication 8, pour la préparation du ramipril.
